Europäisches Patentamt

European Patent Office

Office européen des brevets

(19)

(11) Veröffentlichungsnummer: **0 011 716**
**B1**

(12) **EUROPÄISCHE PATENTSCHRIFT**

(45) Veröffentlichungstag der Patentschrift:
28.03.84

(21) Anmeldenummer: 79104175.9

(22) Anmeldetag: 29.10.79

(51) Int. Cl.³: **G 01 N 33/54**

(54) Reagenz zum Nachweis von infektiöser Mononucleose und Verfahren zu seiner Herstellung.

(30) Priorität: 04.11.78 DE 2847877

(43) Veröffentlichungstag der Anmeldung:
11.06.80 Patentblatt 80/12

(45) Bekanntmachung des Hinweises auf die Patenterteilung:
28.03.84 Patentblatt 84/13

(84) Benannte Vertragsstaaten:
AT BE CH DE FR GB IT LU NL SE

(56) Entgegenhaltungen:
DE - A - 1 598 928
DE - A - 1 920 866
DE - A - 2 516 919
DE - A - 2 546 166
DE - A - 2 659 864
US - A - 3 708 572

JAMA, Preliminary Communication, Oct. 25, 1965, vol. 194, Nr. 4, pages 351-353
Journal of Immunology, 1968, Vol. 101, No. 1, Seiten 18-22

Die Akte enthält technische Angaben, die nach dem Eingang der Anmeldung eingereicht wurden und die nicht in dieser Patentschrift enthalten sind.

(73) Patentinhaber: BEHRINGWERKE AKTIENGESELLSCHAFT, Postfach 1140, D-3550 Marburg/Lahn (DE)

(72) Erfinder: Enders, Burkhard, Dr., Oberer Eichweg 12, D-3550 Marburg/Lahn (DE)

(74) Vertreter: Reuter, Johann-Heinrich, Dr. et al, HOECHST AKTIENGESELLSCHAFT Zentrale Patentabteilung Postfach 80 03 20, D-6230 Frankfurt/Main 80 (DE)

Reagenz zum Nachweis von infektiöser Mononucleose und Verfahren zu seiner Herstellung

Die Erfindung betrifft ein Reagenz zum Nachweis der Infektiösen Mononucleóse, vor allem in Körperflüssigkeiten durch Agglutination, sowie ein Verfahren zu dessen Herstellung.

Die Infektiöse Mononucleose (IM), auch Pfeiffersches Drüsenfieber genannt, ist eine fieberhafte Erkrankung, die bei Kindern und jungen Erwachsenen auftritt. Der Erreger ist nicht gesichert, es wird angenommen, dass Epstein-Barr-Virus (EBV) als ätiologisches Agens fungiert. Bekannt ist, dass während des Ablaufs der Infektion bei bis zu 80% der Patienten heterophile Antikörper auftreten, so dass deren Nachweis von diagnostischem Wert ist. Heterophile Antikörper haben die Eigenschaft, Schafserythrozyten zu agglutinieren, jedoch wird diese Agglutination häufig durch Antikörper gestört, die für die Mononucleose nicht charakteristisch sind. Man war deshalb gezwungen, verhältnismässig komplizierte, zeitraubende Testreihen durchzuführen,um die IM-spezifischen, heterophilen Antikörper zu identifizieren und zu unterscheiden (sog. Differential-Adsorptionsteste).

Es wurde bereits beschrieben, dass die Spezifität des Testes auf Infektiöse Mononucleose durch Verwendung von Pferdeerythrozyten anstelle von Schafserythrozyten erhöht werden könne, doch war auch dieses Verfahren nicht befriedigend. Gemäss der DE-C 1598928 wurde dann ein Reagenz zum Nachweis der Infektiösen Monunucleose beschrieben, bei dem die Pferdeerythrozyten mit Aldehyden behandelt und stabilisiert werden. Dieses Verfahren bringt gegenüber den früher beschriebenen einen Fortschritt im Hinblick auf die Spezifität und Methodik des Testsystems. Es werden jedoch auch damit nicht alle Seren von Trägern IM-spezifischer heterophiler Antikörper als solche erkannt.

Es besteht demnach noch ein Mangel an einem hoch spezifischen und äusserst empfindlichen Testsystem zum Nachweis der Mononucleose.

Es wurde nun gefunden, dass die Behandlung von Pferdeerythrozyten mit Sulfosalicylsäure zu einem spezifisch agglutinierenden Reagenz zum Nachweis der IM Antikörper führt.

Gegenstand der Erfindung ist demnach ein Reagenz zum Nachweis der Infektiösen Mononucleose in Körperflüssigkeiten in einem Agglutinationsverfahren, gekennzeichnet durch von Pferden stammenden Erythrozyten, welche mit Sulfosalicylsäure behandelt und gewünschtenfalls mit einem Farbstoff eingefärbt sind.

Die Behandlung von Erythrozyten, die nicht von Pferden stammen, mit Sulfosalicylsäure wurde von H. Becht in J. Immunol., 1968, S. 18–22, grundsätzlich beschrieben. Erfindungsgemäss werden die Pferdeerythrozyten mit wässriger, 2 bis 10%iger, vorzugsweise 2,5%iger (g/v) Sulfosalicylsäure, insbesondere 5-Sulfosalicylsäure, so lange behandelt, bis die Farbe der Erythrozyten von rot nach braun umschlägt. Dazu werden etwa gleiche Volumenteile einer dichten Erythrozytensuspension von 5 bis 20% (w/v) Erythrozyten in Wasser oder einer geeigneten Pufferlösung mit der Sulfosalicylsäurelösung versetzt. Zweckmässig ist die Pufferung der Erythrozytenlösung sowie der Zusatz von physiologisch verträglichen suspensionsstabilisierenden Substanzen, vor allem geeigneter Polymerer wie Polyvinylpyrolidon oder Dextran, die dem Fachmann allgemein für die Stabilisierung von Erythrozytensuspensionen geläufig sind.

Nach der Sulfosalicylsäurebehandlung der Erythrozyten, die solange durchgeführt wird, bis die Farbe der Suspension von rot nach braun umschlägt, was in der Regel nur wenige Minuten dauert, werden die Erythrozyten durch Sedimentation oder Zentrifugation gewonnen, in einer isotonischen, vorzugsweise gepufferten Salzlösung mehrfach gewaschen, womit das Reagenz dem Grunde nach als fertig anzusehen ist. Zum Nachweis der IM wird die Erythrozytensuspension auf eine Zelldichte von 5 bis $20 \times 10^8$/ml eingestellt.

Für die Aufbewahrung hat es sich als zweckmässig erwiesen, der Lösung stabilisierende Zusätze beizumischen, wie dies für Reagenzien, die aus behandelten Erythrozyten bestehen, grundsätzlich bekannt ist. Man verwendet hierfür in der Biochemie gebräuchliche Neutralsalze, vor allem Kochsalz, Puffermischungen, vor allem Zitrat und Zitronensäure, Kohlenhydrate wie Dextrose oder auch Nucleotide wie Inosin, Adenin oder Guanosin sowie auch antimikrobielle Substanzen wie Chloramphenicol oder Neomycin. Die Lösung wird zweckmässig keinen extremen pH-Werten ausgesetzt, sondern eher um den Neutralpunkt gehalten. Besonders bewährt haben sich pH-Werte zwischen 7,0 und 7,5.

Erythrozyten, die mit einem Aldehyd oder auch mit Sulfosalicylsäure behandelt werden, verlieren die von nativen Erythrozyten her bekannte Brillanz der Farbe. Es liegt deshalb im Sinne der Fortführung des Erfindungsgedanken, die Ablesbarkeit und damit die Verbesserung des Nachweisverfahrens für die IM in der Weise durchzuführen, dass die Sulfosalicylsäure-behandelten Erythrozyten eingefärbt werden. Geeignet gefundene Farbstoffe sind solche, die auch schon bisher dem Fachmann zur Einfärbung von Erythrozyten bekannt waren und auch in der Serologie, Histologie oder Bakteriologie Verwendung finden. Besonders geeignet sind basische Farbstoffe der Triarylamin-, Triarylmethan- und Methin-Reihe wie Kristall-Violett, Malachitgrün, Fuchsin; saure und basische Azofarbstoffe wie Trypanblau und Ponceau-Rot; Phthaleine wie Eosin und Rhodamin; ferner das Coomassie-Blue und die Farbstoffe, wie sie in einer der zahlreichen Modifikationen der Färbung mikroskopischer Präparate nach Ziehl und Neelsen angewandt werden, um nur einige, wesentliche zu nennen.

Besonders deutlich abzulesende Testergebnisse sind erreichbar, wenn das erfindungsgemässe Reagenz mit einem blauen Farbstoff wie z.B. Coomassie-Blue (Anazolenum natricum, WHO) durchgeführt wird.

Die Anfärbung der erfindungsgemäss behandelten Erythrozyten mit einem der beispielhaft genannten Farbstoffe erfolgt nach an sich in der Serologie bekannten Massnahmen. Die Erythrozytensuspension wird mit der Farbstofflösung in einer der dafür üblichen Konzentrationen versetzt und der Verlauf der Anfärbung beobachtet. Erscheint die Farbintensität ausreichend, werden die Erythrozyten durch Sedimentation oder Zentrifugation von der Farbstofflösung getrennt, die Erythrozyten mehrfach in der oben beschriebenen Weise gewaschen, mit stabilisierenden Zusätzen versetzt und schliesslich zu einer Suspension enthaltend von 5 bis $20 \times 10^8$ Erythrozyten/ml resuspendiert.

Die Aufbewahrung des Reagenzes erfolgt sowohl für die gefärbten wie für die ungefärbten Erythrozyten zweckmässig bei einer Temperatur unterhalb der Raumtemperatur, jedoch oberhalb von 0°C, vor allem bei etwa +3 bis +6°C.

Das diagnostische Verfahren zum Nachweis der IM besteht darin, dass das auf heterophile Antikörper verdächtige Serum in einer Verdünnungsreihe mit einer konstanten Menge der erfindungsgemäss behandelten Erythrozyten versetzt und die gegebenenfalls auftretende Agglutination registriert wird. Die Agglutination wird zweckmässig auf Glasobjektträgern oder grösseren, in einzelne Kompartimente unterteilte Platten durchgeführt. Besonders gut lässt sich der Test auch in den bekannten Tüpfelplatten durchführen. Dabei zeigen sich die gefärbten Erythrozyten besonders brillant in ihrer Agglutination und ermöglichen eine im Vergleich zu den nicht gefärbten bessere Ablesemöglichkeit.

Ein positives Ergebnis auf IM liegt vor, wenn eine makroskopisch sichtbare, grobe Agglutination, eine sogenannte Schollenbildung sichtbar wird. In Zweifelsfällen erleichtert die Verwendung einer Lupe oder eines Plattenmikroskops die Erkennung der Agglutination. Ein negatives Ergebnis ist gekennzeichnet durch eine feinkörnig homogen bleibende Suspension der Testerythrozyten.

Die Erfindung wird am nachstehenden Beispiel näher erläutert:

Beispiel

A. Behandlung von Pferdeerythrozyten

350 ml frisches Pferdeblut in Alsevers-Lösung (1:1) werden zentrifugiert und 5 mal jeweils 10 Minuten lang in der 10-fachen Menge (bezogen auf Volumen) des Erythrozytensediments in phosphatgepufferter Kochsalzlösung (PBS), pH 7,2, gewaschen. Die Zentrifugation wird bei 3000 U/min durchgeführt. Inzwischen werden 15 g Polyvinylpyrrolidon (PVP; MG 350000) in 1500 ml phosphatgepufferter NaCl-Lösung (PBS, pH 7,2) gelöst. Nach dem letzten Erythrozytenwaschgang wird das Sediment durch Zugabe der 1%igen PVP-PBS-Lösung auf eine 5 bis 10%ige Erythrozytensuspension eingestellt.

Unter tropfenweiser Zugabe des gleichen Volumens einer 2,5%igen 5-Sulfosalicylsäure-Lösung in 1% PVP-Lösung und unter ständigem Rühren (Magnetrührer) für 30 bis 40 Minuten bei Zimmertemperatur erfolgt die Umsetzung mit den Erythrozyten. Der Farbumschlag von rot nach braun vollzieht sich sofort nach vollständiger Zugabe der Sulfosalicylsäure-PVP-PBS-Lösung. 30 Minuten nach erfolgter Verfärbung der Erythrozytensuspension werden die Erythrozyten 5 mal in PBS, pH 7,2, gewaschen und mittels Zentrifugation (3000 U/20 min. pro Waschung) gewonnen.

B. Färbung der sulfosalicylsäurebehandelten Erythrozyten

Zur Herstellung einer rotgefärbten Suspension wird als Farblösung Ziehl-Neelsens-Karbolfuchsinlösung (ZN-Lsg., Merck 9215) verwendet. Die Anfärbung der sulfosalicylsäurebehandelten Erythrozyten wird im Anschluss an die 2. Waschung der Erythrozytensediments nach A) durchgeführt. Dazu wird das Sediment in der ZN-Farblösung aufgenommen, die aus der ZN-Lösung durch eine 1:30 Verdünnung mit PBS, pH 7,2, hergestellt wurde, und 15 Minuten bei Raumtemperatur inkubiert.

Danach folgen noch 3 Waschungen in PBS, pH 7,2, wie in A beschrieben. Der letzte Waschüberstand ist wasserklar. Durch Zugabe der Stabilisatorlösung C) zum letzten Sediment wird eine 10%ige rotgefärbte Erythrozytensuspension hergestellt. Sie wird bis zur Weiterverarbeitung im Kühlschrank aufbewahrt.

C. Stabilisatorlösung, pH 7,2

Folgende Substanzen werden in 1000 ml dest. Wasser gelöst:

| | |
|---|---|
| 20,5 g/l | Dextrose, wasserfrei |
| 8,0 g/l | Na-Zitrat $3 H_2O$ |
| 4,2 g/l | NaCl |
| 0,55 g/l | Zitronensäure |
| 0,429 g/l | Chloramphenicol |
| 0,122 g/l | Neomycinhydrochlorid (832 mg Neomycin B – Base pro g) |
| 4,0 g/l | Inosin |
| 0,2 g/l | Adensin |
| 0,2 g/l | Guanosin |
| 0,1 g/l | Thimerosal |

Die Lösung wird mit 5 n NaOH auf pH 7,3 eingestellt und anschliessend sterilfiltriert.

D. Herstellung des IM-Reagenzes

Zur Verwendung des IM-Reagenzes auf Glasobjektträgern bzw. Glasagglutinationsplatten wird eine 7%ige Erythrozytensuspension in der Stabilisatorlösung C) hergestellt. Diese Zellkonzentration gilt sowohl für ungefärbte als auch gefärbte Erythrozyten.

Bei Verwendung von Porzellan-Tüpfelplatten erweist sich eine 3,5%ige Zellkonzentration in C) als optimal. Gefärbte Erythrozyten zeigen eine deutlichere Agglutinationsbildung und damit eine bessere Ablesemöglichkeit der Reaktion. Die Abfüllung des Reagenzes erfolgt in Fläschchen mit Schraubkappen und Tropfpipette mit einem

Tropfvolumen von ca. 35 µl. Das Reagenz wird bei +4° bis +6°C aufbewahrt.

E. Durchführung des IM-Nachweises

Ein Tropfen Patientenserum (35 µl) wird mit der Pipette in die Mitte eines Feldes einer Testplatte aufgetropft und mit einem Tropfen IM-Reagenz (D) mit Hilfe eines sauberen Rührstäbchens vorsichtig vermischt. Dann lässt man die Testplatte für ca. 2 Minuten leicht rotieren.

Ein positives Ergebnis auf infektiöse Mononucleose liegt vor, wenn eine makroskopisch sichtbare, grobe Agglutination (Schollenbildung am Rand) sichtbar wird. Zur Entscheidung in Zweifelsfällen kann eine Lupe oder ein Plattenmikroskop verwendet werden.

Ein negatives Ergebnis ist gekennzeichnet durch ein Fehlen eines Agglutinationsmusters. Die Suspension bleibt feinkörnig homogen.

Seren von 107 gesicherten IM-Fällen waren zu 88,8% mit dem erfindungsgemässen Reagenz positiv.

**Patentansprüche**

1. Reagenz zum Nachweis der Infektiösen Mononucleose in Körperflüssigkeiten in einem Agglutinationsverfahren mittels Pferdeerythrozyten, dadurch gekennzeichnet, dass die Erythrozyten mit Sulfosalicylsäure behandelt sind.

2. Reagenz nach Anspruch 1, dadurch gekennzeichnet, dass die Erythrozyten mit einem Farbstoff eingefärbt sind.

3. Verfahren zur Herstellung eines Reagenzes nach Anspruch 1 oder 2, dadurch gekennzeichnet, dass einer 5 bis 20%igen wässrigen Suspension von Pferdeerythrozyten soviel einer 2 bis 10%igen wässrigen Lösung von Sulfosalicylsäure zugesetzt wird, dass die Farbe nach braun umschlägt und die Suspension sodann gegebenenfalls mit einem Farbstoff eingefärbt wird.

**Claims**

1. Reagent for detecting infectious mononucleosis in body fluids by agglutination of horse erythrocytes which comprises erythrocytes treated with sulfosalicylic acid.

2. Reagent according to claim 1 which comprises erythrocytes colored with a dyestuff.

3. Process for the manufacture of a reagent as claimed in claims 1 or 2, which comprises adding to a 5 to 20% aqueous suspension of horse erythrocytes a 2 to 10% aqueous solution of sulfosalicylic acid in an amount such that the color turns from red to brown and then optionally coloring the erythrocytes with a dyestuff.

**Revendications**

1. Réactif pour le diagnostic de la mononucléose infectieuse dans les fluides corporels dans un procédé d'agglutination avec des érythrocytes de cheval, caractérisé en ce que les érythrocytes sont traités avec de l'acide sulfosalicylique.

2. Réactif selon la revendication 1, caractérisé en ce que les érythrocytes sont colorés avec un colorant.

3. Procédé pour la préparation d'un réactif selon l'une des revendications 1 ou 2, caractérisé en ce qu'à une suspension aqueuse à de 5 à 20% d'érythrocytes de cheval, on ajoute une solution aqueuse à de 2 à 10% d'acide sulfosalicylique jusqu'à ce que la couleur vire au brun et on colore éventuellement ensuite la suspension avec un colorant.